# FASCICULE DE BREVET EUROPEEN

(11) **EP 0 700 293 B1**
(45) Date de publication et mention de la délivrance du brevet: **04.03.1998**
(21) Numéro de dépôt: 94917056.7
(22) Date de dépôt: 25.05.1994
(51) Int. Cl.: A61K 31/565

(54) **DIHYDROTESTOSTERONE POUR ANDROGENOTHERAPIE**
DIHYDROTESTOSTERON FÜR ANDROGENTHERAPIE
DIHYDROTESTOSTERONE FOR USE IN ANDROGENOTHERAPY

(30) Priorité: 25.05.1993 FR 9306224
(43) Date de publication de la demande: 13.03.1996
(73) Titulaire: Laboratoires BESINS ISCOVESCO Société anonyme dite :, 75003 Paris (FR)
(72) Inventeur: DE LIGNIERES, Bruno, F-91210 Draveil (FR)
(74) Mandataire: Bruder, Michel
(86) Numéro de dépôt international: FR9400617
(87) Numéro de publication internationale: WO9427609

(56) Documents cités:
- THE PROSTATE, vol.15, no.2, 1989 pages 95 - 103 'Prevention and treatment of experimental prostate cancer in Lobound-Wistar rats. I. Effects of Estradiol, Dihydrotestosterone, and Castration'
- THE PROSTATE, vol.10, no.4, 1987 pages 325 - 331 'Dihydrotestosterone does not induce prostate adenocarcinoma in L-W rats'
- BULL.CANCER, vol.73, no.1, 1986 pages 36 - 46 'Cancer de la prostate: bases biologiques pour l'emploi d'un antiandrogène dans le traitement'
- THE PROSTATE, vol.6, no.1, 1985 pages 19 - 25 'DHT in prostate cancer tissue- a guide to management and therapy'
- Impact Médecin Hebdo n 203, 10.09.93, p.75
- Scrip n 1867 du 26.10.93, p.20
- Hypertrophie béningne de la prostate
- Le Point JIM/253, pp.39-43
- The Prostate vol.22, 1993, pp.43-51
- The New England J. of Medicine, vol.327, 22.10.92, pp.1185-1191
- J. of Andrology vol.12, n 6, 1991, p.372

## Description

La présente invention concerne des formes d'administration de la dihydrotestostérone, en abrégé DHT, dont les emplois thérapeutiques sont multiples, et en particulier des formes destinées à l'androgénothérapie, notamment chez des sujets à risque d'hyperplasie de la prostate, en particulier d'hyperplasies bénignes.

Il avait été jusqu'ici suggéré que, de l'emploi de la DHT pourraient résulter des risques pour la prostate de sujets de plus de cinquante ans. Le cancer prostatique et l'hypertrophie prostatique bénigne, en abrégé HPB, sont des désordres qui dépendent en partie des androgènes et l'on conçoit aisément que ce genre de risque est à prendre très sérieusement en considération, notamment, dans le cas du traitement de l'andropause. A partir de 60 ans, l'HPB, par comparaison avec le cas de jeunes adultes, devient une situation statistiquement normale chez les sujets non traités. La première période de croissance prostatique est liée à la puberté et à la sécrétion testiculaire d'androgènes, mais les influences hormonales sur la seconde période de croissance prostatique après 50 ans sont jusqu'à présent loin d'être identifiées avec précision.

Or, l'expérience a montré qu'une seconde augmentation rapide de la sécrétion d'androgènes testiculaires est nettement exclue et qu'au contraire, les niveaux plasmatiques des androgènes biodisponibles tendent à diminuer après 50 ans. Certains auteurs ont proposé comme explication plausible l'accumulation de DHT dans le tissu prostatique, mais cette supposition a été écartée sur la base d'études plus récentes comparant les tissus prostatiques d'origines similaires avec ou sans HPB.

Des taux normaux, voire bas, de testostérone (en abrégé T) ou de DHT, dans le plasma et le tissu prostatique sont compatibles avec le développement d'une HPB, et des régressions limitées du volume prostatique ne sont décrites que pour une suppression quasi totale de la testostérone plasmatique en circulation ou de la DHT dans le tissu prostatique, obtenus grâce à plusieurs agents pharmacologiques.

Une diminution de plus de 80% de la concentration tissulaire prostatique de la DHT par inhibition à la 5 alpha-réductase n'induit qu'une diminution moyenne de 18% du volume de la prostate; et une quasi annulation du taux plasmatique de la testostérone réduit à une moyenne de 0,1 ng/ml (c'est-à-dire 50 fois inférieur aux taux physiologiques) est nécessaire pour induire une régression de 30 à 40% du volume prostatique.

On rappelera que, par exemple la DHT présente un noyau A saturé dans sa molécule alors que, par exemple, le 17 béta-oestradiol présente un noyau A benzénique, c'est-à-dire insaturé aromatique. L'aromatase dont il sera question ci-après et dont la testostérone est le substrat, favorise la transformation de noyaux totalement ou partiellement saturés en noyaux aromatiques totalement insaturés. Quant à l'alpha-réductase, en induisant les réactions de réduction des insaturations, permet la formation de la DHT à partir de la testostérone.

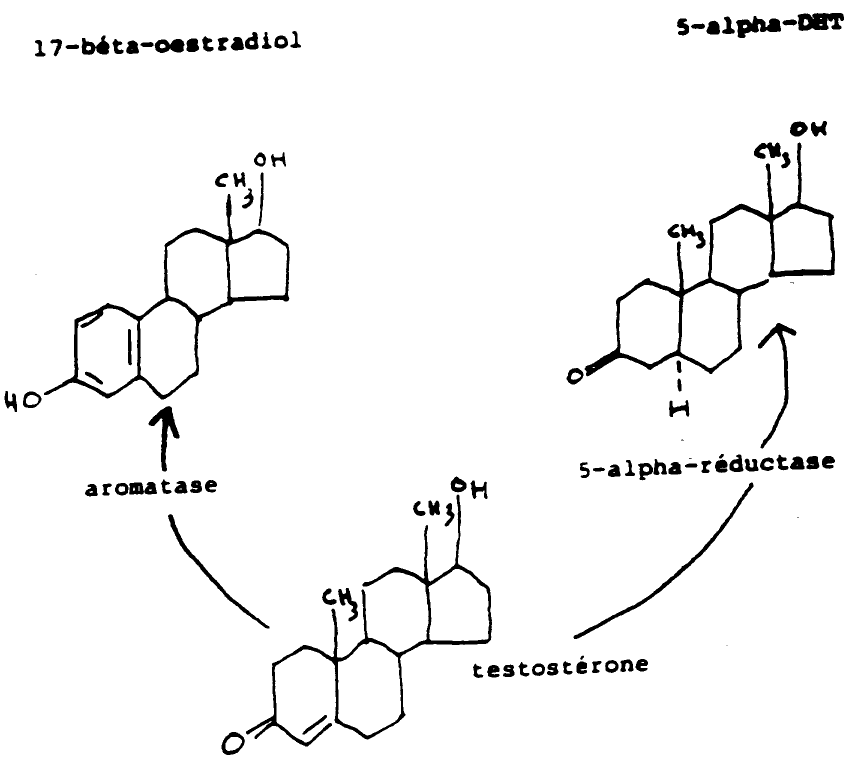

Les constatations mentionnées plus haut, ne confirment pas l'hypothèse selon laquelle toute augmentation spontanée de la stimulation, par la T ou la DHT, des cellules prostatiques épithéliales ou stromales, puisse constituer la cause initiale réelle de l'HPB chez l'homme âgé. Elles ne fournissent d'ailleurs pas d'informations utiles sur les risques et profits, pour la prostate, d'une augmentation compensatoire modérée de la stimulation androgénique chez l'homme âgé.

Comme aucun accroissement, au-delà des normes physiologiques, dans la stimulation par la T ou la DHT de la prostate ne peut expliquer l'HPB, on a proposé d'autres hypothèses.

L'une d'elles prend en considération le changement d'équilibre, en fonction de l'âge, entre oestrogènes et androgènes. Des études expérimentales ont démontré l'inaptitude des androgènes à cycles A saturés et non aromatisables en oestrogènes (à noyau A totalement insaturé) d'induire un stade initial d'hypertrophie prostatique. Des androgènes aromatisables tels que la testostérone et l'androstènedione peuvent induire des modifications hyperplasiques de la prostate de singes, mais ces effets sont inversés par addition d'un inhibiteur d'aromatase.

De même, quatorze mois de traitement par la DHT non aromatisable, de rats LW mâles susceptibles d'un cancer de la prostate, n'ont pu induire ni une hypertrophie macroscopique, ni une microscopique, ni un cancer de la prostate. A l'inverse, des rats traités avec des doses équivalentes de testostérone ont montré une incidence accrue d'hypertrophie bénigne, et un adénocarcinome a été détecté chez 24% des animaux traités, avec des tumeurs additionnelles in situ pour 16%. Parmi le groupe de référence non traité, on a observé une situation intermédiaire pour quelques cas d'HPB et quelques néoplasmes in situ, mais sans adénocarcinome envahissant.

De telles expériences amènent à penser que, chez l'animal à haut risque de désordre prostatique, la T et la DHT présentent des influences très différentes, un long traitement par la testostérone aromatisable augmentant l'incidence, à la fois sur les désordres prostatiques malins ou bénins, tandis que le traitement par la DHT non aromatisable diminue ces deux risques. Une étude à court terme avec un inhibiteur d'aromatase chez l'homme souffrant d'HPB a montré une réduction moyenne de 26% du volume de la prostate, ce qui renforce l'hypothèse de la responsabilité, également pour l'homme, de certains oestrogènes.

Tout permet de penser qu'il existe un effet des oestrogènes sur la prostate. On a pu identifier les récepteurs d'oestradiol dans la prostate humaine normale ou dans au stade initial d'HPB pour des concentrations plus élevées chez les cellules stromales que chez les épithéliales.

On a identifié l'activité de l'aromatase susceptible de synthétiser localement des oestrogènes à partir de substrats comme la testostérone ou l'androstène-dione, dans la partie stromale de la prostate.

Des concentrations modérées d'oestrogènes en présence d'androgènes stimulent de façon spécifique la croissance stromale à court terme, mais à moyen et à long terme, les cellules stromales influencent la croissance épithéliale par des effets paracrines, tandis que les androgènes, et en particulier la DHT, influencent principalement la croissance épithéliale et maintiennent un équilibre physiologique entre stroma et épithélium.

Non seulement l'activité de l'aromatase tend à augmenter avec l'âge, mais l'expérience laisse supposer une légère tendance vers une moindre sensibilité des cellules prostatiques à la DHT avec l'âge, et au contraire, vers une plus forte sensibilité à l'oestradiol.

La stimulation par l'oestradiol peut relativement augmenter en fonction de la montée des niveaux plasmatiques des oestrogènes, de l'aromatisation de la testostérone des tissus et de la réceptivité potentielle des cellules stromales due aux changements autocrines/paracrines.

Dans un premier temps, le stroma prolifère, créant des nodules croissants qui influenceront, dans un second temps, des éléments glandulaires par stimulation paracrine. Dans un troisième temps, les facteurs autocrines/paracrines maintiendront les cellules stromales et épithéliales sous contrôle, la partie la plus agressivement croissante du tissu devenant indépendante de toute stimulation stéroïde.

On peut donc en conclure que ni la DHT seule, ni l'oestradiol seul ne peuvent surstimuler les cellules stromales ou épithéliales prostatiques.Chez l'homme d'âge moyen, les modifications de la prostate semblent résulter d'une augmentation progressive du rapport oestradiol/DHT dans le stroma.

Dans l'art antérieur, la plupart des manipulations hormonales se sont orientées vers une suppression des androgènes et n'ont entraîné de bénéfices objectifs que lorsque la stimulation androgénique a été réduite à un niveau proche de zéro. Une telle situation ne répond pas au besoin d'une substitution hormonale andropausique.

De plus, un traitement anti-androgénique peut supprimer plus de cellules normales dépendant des androgènes que de cellules stimulées par des oestrogènes, potentiellement nocives à long terme; son avantage pourrait être limité dans le temps.

Par ailleurs, un traitement à la DHT, qui, contrairement à celui à la testostérone, inclut à la fois une activité androgénique et antiaromatisation, est assez séduisant pour son aptitude théorique possible à accroître la stimulation androgénique tout en empêchant on en réduisant considérablement la tendance, avec l'âge, à l'augmentation du rapport oestradiol/DHT. La diminution des oestrogènes, contrairement à celle des androgènes, ne présente pas d'effet secondaire appréciable chez l'homme âgé.

Selon une troisième hypothèse, on considère que la forte augmentation avec l'âge des globulines de liaisons aux hormones sexuelles (en abrégé GLHS, et en version anglaise sex hormone binding globulin, soit SHBG) constitue la meilleure explication pathogène aux désordres prostatiques.

Ce type de globuline constitue le véhicule des hormones sexuelles. Un excès de GLHS, non liée à des stéroïdes, est susceptible de se lier à certains récepteurs des membranes cellulaires prostatiques, et de déclencher des effets stimulants sur la croissance cellulaire.

La croissance in vitro, de cellules de carcinome humain est stimulée par addition de GLHS au milieu de culture. Une addition supplémentaire de testostérone ne modifie pas ou renforce l'effet de la GLHS.

A l'inverse, l'addition de DHT à haute concentration réduit cet effet de stimulation. La saturation des sites à haute affinité de la GLHS plasmatique par la DHT empêche la liaison de la GLHS aux récepteurs des membranes cellulaires.

Cette dernière hypothèse est compatible avec la précédente car l'augmentation de la GLHS semble liée à une augmentation en fonction de l'âge de l'activité de l'aromatase dans le foie, et que par ailleurs cela suggère également un avantage potentiel dans l'utilisation de la DHT par rapport à la testostérone.

Enfin, l'instabilité dans la stimulation androgénique peut revêtir une importance pathogénique, car une stimulation androgénique intermittente de la prostate stimule expérimentalement plus de mitose dans l'épithélium de la prostate qu'une concentration constante et stable des androgènes.

On peut donc penser d'après ces études antérieures, que des compositions de testostérone à action prolongée, avec de grandes variations des androgènes plasmatiques sont moins qu'optimales pour des études in vivo sur la prostate humaine.

Pour résumer, on peut dire que l'on a proposé essentiellement dans l'art antérieur :
a) de diminuer le niveau d' activité de la 5 alpha-réductase dans la prostate d'où un effondrement du taux de DHT,
b) de recourir à une antiaromatase et de produire une chute des concentrations d'oestrogènes.

On a souligné les inconvénients de ces méthodes qui ne permettent en aucun cas le rétablissement d'une stimulation androgénique physiologique.

L'administration de DHT sous forme de gel percutané ou d'heptanoate injectable par voie intramusculaire n'a été envisagée selon l'art antérieur que pour le traitement de gynécomastie ou d'hypogonadisme. Mais il est évident que les bénéfices et les risques, en particulier, prostatiques, chez les adolescents et les jeunes adultes sont différents de ceux de l'homme âgé ou, plus généralement, de plus de 50 ans. On pourra se reporter sur ce type d'administration à la demande de brevet européen 0.197.753 à priorité du 30 mars 1984 déposé au nom du Baylor College of Medicine.

Selon la présente invention, on recourt à l'effet antiaromatisation lié à l'augmentation du taux de DHT. Mais alors que l'art antérieur considérait que la DHT était nocive dans le sens de l'hypertrophie prostatique, l'invention est basée sur l'utilisation de concentrations plasmatiques plus élevée attestées par des dosages précis, qui donnent l'effet inverse de celui jusque là attendu. Cet effet contraire aux considérations habituelles n'avait jamais été décrit et, se révèle comme on le verra plus loin, efficace.

Par ailleurs, selon un mode de réalisation préféré de l'invention, les compositions sont administrées par voie percutanée et donc distribuant les principes actifs de façon plus régulière et mieux prolongée dans le temps, que dans le cas des injections qui provoquent de fortes variations intraindividuelles de principe actif, comme dans le cas de la demande européenne citée ci-dessus.

Il convient pour l'homme de l'art d'administrer des quantités de DHT qui assurent une teneur plasmatique de l'ordre de 2,5 à 10 ng/ml. Les doses administrées dépendront donc de la teneur initiale chez le patient et de la teneur souhaitée. Des analyses sanguines permettent de suivre l'évolution et de faire varier les doses administrées en conséquence.

On notera également que la somme des teneurs plasmatiques en T + DHT est en moyenne supérieure à 3,5 ng/ml chez l'homme normal. La teneur en testostérone est, en moyenne, dix fois plus forte que celle en DHT. Selon l'invention, on réduit cette proportion et on va même jusqu'à l'inverser, et selon l'invention on se fixera pour but préférentiel plus de 3 ng/ml de DHT pour moins de 1,5 ng/ml de testostérone.

Pour mieux faire comprendre les caractéristiques techniques et les avantages de la présente invention, on va en décrire un exemple de réalisation étant bien entendu que celui-ci n'est pas limitatif quant à son mode de mise en oeuvre et aux applications qu'on peut en faire.

On réalise la composition suivante constituée par un gel hydroalcoolique à teneur en DHT de 0,5 à 3,5%, de préférence 2,5%.

On administre 5 à 10 g par jour de ce gel par voie percutanée selon les besoins appliqués sur une grande surface (bras, avant-bras, épaules). On contrôle les taux plasmatiques en T et en DHT, et on corrige la dose journalière en conséquence pour maintenir ces taux dans les limites ci-dessus indiquées.

On a effectué les essais comparatifs suivants :

Au cours d'études menées par la demanderesse, on a suivi les évolutions prostatiques chez 37 hommes âgés de 55 à 70 ans avec de hauts niveaux plasmatiques de GLHS et des symptômes cliniques attribués à un hypogonadisme, et ceci avec un traitement à la DHT par voie percutanée et pendant une durée de 6 mois à 5 années.

Chez 27 sujets dont on contrôlait les niveaux de DHT plasmatique, de façon à éventuellement moduler les doses administrées, on a augmenté lesdits niveaux jusqu'à 2,5 à 6 ng/ml. Il en est résulté une diminution des gonadotrophines ainsi que des teneurs plasmatiques en testostérone qui sont passées à moins de 1,5 ng/ml (de 0,5 à 1,4 selon les cas) ; quant aux niveaux plasmatiques d'oestradiol, ils ont diminué de 50%.

Parmi ce groupe de sujets, le volume de la prostate a diminué de façon significative, comme on a pu l'évaluer par ultrasons et par PSA (Prostate Specific Antigen). Le volume moyen des prostates était de 31,09+/-16,31 grammes avant traitement et de 26,34+/-12,72 grammes, soit une réduction moyenne de 15,4%, après un traitement d'une durée moyenne de 1,8 année à la DHT (P=0,01).

Par contre, dans un groupe de 10 hommes avec des teneurs plasmatiques plus basses en DHT, c'est-à-dire inférieures à 2,5 ng/ml, que l'on a traité avec les mêmes compositions sans tenir compte des variations des niveaux plasmatiques et donc sans modulation des doses administrées, on a constaté une moindre diminution des teneurs plasmatiques en testostérone qui restent au-dessus de 2 ng/ml et la non-variation des teneurs plasmatiques en oestradiol, avec une légère augmentation non significative du volume de la prostate qui est passée de 31,6+/-16,38 grammes avant traitement à 36,15+/-16,62 grammes après un traitement d'une durée moyenne de 1,7 année, soit +14,4% d'augmentation.

## Revendications

1. Composition androgénothérapique à effet favorable sur l'hyperplasie de la prostate, caractérisée par le fait qu'elle contient de la dihydrotestostérone en quantité administrée permettant d'atteindre un taux plasmatique de la dihydrotestostérone seule supérieur à 2,5 ng/ml et à un taux plasmatique total en testostérone et en dihydrotestostérone supérieur à 3,5 ng/ml chez l'homme normal.

2. Composition selon la revendication 1, caractérisée par le fait qu'elle se présente sous forme administrable par voie percutanée.

3. Composition selon l'une des revendications 1 ou 2, caractérisée par le fait qu'elle est constituée par un gel hydroalcoolique à 0,5 à 3,5% de dihydrotestostérone.

## Claims

1. Androgenotherapeutic composition having a favourable effect on the hyperplasia of the prostate, characterized in that it contains dihydrotestosterone in an administered quantity making it possible to attain a plasma level of the dihydrotestosterone alone greater than 2.5 ng/ml and a total plasma level in testosterone and in dihydrotestosterone greater than 3.5 ng/ml in the normal man.

2. Composition according to Claim 1, characterized in that it is presented in a form that may be administered by the percutaneous route.

3. Composition according to Claims 1 or 2, characterized in that it is constituted by a water alcohol gel with 0.5 to 3.5% of dihydrotestosterone.

## Patentansprüche

1. Androgentherapeutische Zusammensetzung mit günstiger Wirkung auf die Hyperplasie der Prostata, dadurch gekennzeichnet, daß sie Dihydrotestosteron in einer Menge enthält, die bei Verabreichung bei einem normalen Mann die Einstellung eines Plasmaspiegels von Dihydrotestosteron allein oberhalb von 2,5 ng/ml erlaubt und eines Plasmaspiegels an Testosteron und Dihydrotestosteron insgesamt oberhalb von 3,5 ng/ml.

2. Zusammensetzung nach Anspruch 1, dadurch gekennzeichnet, daß sie in perkutan verabreichbarer Form vorliegt.

3. Zusammensetzung nach einem der Ansprüche 1 oder 2 dadurch gekennzeichnet, daß sie durch ein hydroalkoholisches Gel mit 0,5 bis 3,5 % Dihydrotestosteron gebildet wird.
